# EUROPEAN PATENT SPECIFICATION

(11) **EP 1 485 343 B1**
(45) Date of publication and mention of the grant of the patent: **23.12.2009**
(21) Application number: 02717024.0
(22) Date of filing: 18.03.2002
(51) Int. Cl.: C07C 69/732, C07D 207/34

(54) **AMORPHOUS HMG-COA REDUCTASE INHIBITORS OF DESIRED PARTICLE SIZE**
AMORPHE HMG-COA-REDUKTASE-INHIBITOREN MIT GEWÜNSCHTER TEILCHENGRÖSSE
INHIBITEURS DE HMG-COA REDUCTASE AMORPHE AVEC TAILLE SOUHAITEE DES PARTICULES

(43) Date of publication of application: 15.12.2004
(73) Proprietor: Biocon Limited, Bangalore 561 229 (IN)
(72) Inventor: POORNAPRAJNA, Acharya, Hebbagodi, Bangalore 561 229 (IN); MATHEW, Joy, Hebbagodi, Bangalore 561 229 (IN); CHANDRAPPA, Ravindra, Hebbagodi, Bangalore 561 229 (IN); SRIDHARAN, Madhavan, Hebbagodi, Bangalore 561 229 (IN); GANESH, Sambasivam, Hebbagodi, Bangalore 561 229 (IN)
(74) Representative: Robson, Aidan John
(86) International application number: PCT/IN2002/000045
(87) International publication number: WO 2003/078379

(56) References cited:
- WO-A-97/03960
- WO-A1-00/53566
- WO-A1-01/10813
- WO-A1-01/28999
- WO-A1-01/42209
- WO-A1-97/03958
- US-A- 4 346 227

## Description

### FIELD OF THE INVENTION

The present invention relates to a process for the production of amorphous HMG-CoA reductase inhibitors of desired particle size.

### BACKGROUND OF THE INVENTION

Lovastatin, pravastatin, simvastatin, mevastatin, atorvastatin, fluvastatin and cervastatin and derivatives and analogs thereof are known as HMG-CoA reductase inhibitors and are used as antihypercholesterolemic agents. The majority of them are produced by fermentation using microorganisms of different species identified as species belonging to *Aspergillus, Monascus, Nocardia, Amycolatopsis, Mucor or Penicillium* genus, some are obtained by treating the fermentation products using the methods of chemical synthesis or they are the products.

The present invention relates to amorphous form HMG-CoA reductase inhibitors, which are useful as a pharmaceutical substance, to the method for its production and isolation HMG-CoA reductase inhibitors, are used for the treatment of hyperlipidemia and hypercholesterolemia, both of which are risk factors for arteriosclerosis and coronary heart disease.

United States Patent 5,273,995, describes that R-form of the ring opened acid form inhibits the biosynthesis of cholesterol. Atorvastatin in its calcium salt form, i.e. amorphous [R-(R*,R*)]-2-(4-fluorophenyl)-β,δ-dihydroxy-5-(1-methylethyl)-3-phenyl-4-[(phenylamino)carbonyl]-1H-pyrrole-1-heptanoic acid hemi calcium salt (2: 1) is discussed in literature.

Various United States patents like, 5,003,080; 5,097,045; 5,103,024; 5,124,482; 5,149,837; 5,248,793; 5,280,126; 5,342,952, describe various processes and key intermediates for preparing atorvastatin calcium.

The process mentioned in the above patents does not produce atorvastatin calcium in its amorphous form consistently. Often a mixture of crystalline and amorphous form is obtained which is not suitable for filtration and drying and therefore not a desirable process for large-scale production.

PCT application, WO 97/03959, discloses novel crystalline forms of atorvastatin calcium designated as Form I, Form II, and Form IV and method for their preparation. PCT application WO 97/03960 describes a procedure for converting the crystalline form of atorvastatin to the amorphous form.

The process described in the above mentioned patent involves dissolving the crystalline atorvastatin (form-I) in a non hydroxylic solvent like tetrahydrofuran or mixtures of tetrahydrofuran and toluene, followed by removal of the solvents under high temperature (about 90°C) and high vacuum (about 5mm). This process may not suitable on a large scale as the conditions used for drying may lead to degradation of the product.

PCT application WO 00/71116 claims a process for the preparation of amorphous atorvastatin calcium where the crystalline form is dissolved in a non-hydroxilic solvent is treated with a non-polar hydrocarbon anti-solvent followed by the removal of the solvent to result in the amorphous form.

Pravastatin, which was first reported as a metabolite of compactin in US4,346,227. WO01/43723 describes certain novel forms of pravastatin which are characterized by X-Ray patterns.

### SUMMARY OF THE INVENTION

It is desirable to have a process, which provides amorphous HMG-CoA reductase inhibitor using a procedure, which can be readily scaled up to a commercial scale. The present invention describes a process, which is ideal for large scale production of amorphous HMG-CoA reductase inhibitor.

The present invention provides a process for the preparation of amorphous atorvastatin or pravastatin and hydrates thereof of desired particle size, which comprises:
(i) dissolving the heterogeneous mixture of HMG-CoA reductase inhibitor in a hydroxylic solvent and
(ii) removing the solvent to obtain amorphous HMG-CoA reductase inhibitor.
The process may further comprise
(iii) subjecting the amorphous atorvastatin or pravastatin to milling.

The solvent is removed by freeze drying or spray drying.

The amorphous atorvastatin or pravastatin has a particle size of 1 to 150 microns.

The hydroxylic solvent solvent in step (i) is methanol.

Major advantages of the present invention compared to the prior art processes are:
i. Produces amorphous HMG-CoA reductase inhibitor consistently.
ii. Results in the final product of desired particle size.
iii. Avoids the necessity to remove solvents.
iv. Simpler and faster filtration.
v. Easy to operate on large-scale.
vi. Avoids the use of hydrocarbons.

The present invention thus provides a simple and novel process for the preparation of amorphous atorvastatin calcium and hydrates thereof. The starting material used in the instant invention comprises of a mixture of both amorphous and crystalline forms - henceforth referred to as heterogeneous mixture.

### BRIEF DESCRIPTION OF THE ACCOMPANYING FIGURES

Figure 1 is the diffractogram of amorphous atorvastatin calcium. The horizontal axis represents 2θ and the vertical axis corresponds to peak intensity.
Figure 2 is the diffractogram of amorphous pravastatin. The horizontal axis represents 2θ and the vertical axis corresponds to peak intensity.

### DETAILED DESCRIPTION OF THE INVENTION

Major advantages of the present invention compared to the prior art processes are:
i. Produces amorphous HMG-CoA reductase inhibitor consistently.
ii. Results in the final product of desired particle size.
iii. Avoids the necessity to remove solvents.
iv. Simpler and faster filtration.
v. Easy to operate on large-scale.
vi. Avoids the use of hydrocarbons.

The present invention thus provides a simple and novel process for the preparation of amorphous atorvastatin calcium and hydrates thereof. The starting material used in the instant invention comprises of a mixture of both amorphous and crystalline forms - henceforth referred to as heterogeneous mixture.

The present invention has been described in terms of its specific embodiments, certain modifications and equivalents will be apparent to those skilled in the art and are intended to be included within the scope of the present invention.

### EXAMPLES

### Example 1

### [R-(R*,R*)]-2-(4-fluorophenyl)-β,δ-dihydroxy-5-(1-methylethyl)-3-phenyl-4-[(phenylamino)carbonyl]-1H-pyrrole-1-heptanoic acid hemi calcium salt (Amorphous Atorvastatin calcium).

A heterogenous mixture of atorvastatin (5g) was added to methanol (100ml) and the resulting reaction mixture was freeze dried to afford amorphous atorvastatin.

The particle size as measured by malvern particle size analyser gave the following pattern:

| | |
|---|---|
| D₁₀ | 3 microns |
| D₅₀ | 9 microns |
| D₉₀ | 16 microns |

### Example 2

### [1S-([1α(β*,δ*)2α,6α,8B(R*),8aα]]-1,2,6,7,8,8a-hexahydro-β,δ,6-trihydroxy-2-methyl-8-(2-methyl-1-oxobutoxy)-1-naphthalene heptanoic acid sodium salt (Amorphous Pravastatin)

A heterogenous mixture of pravastatin sodium (5g) was added to methanol (100ml) and the resulting reaction mixture was spray dried to afford amorphous pravastatin sodium. The amorphous pravastatin sodium so obtained was milled using an air jet mill

The particle size as measured by malvern particle size analyser gave the following pattern:

| | |
|---|---|
| D10 | 3 micron |
| D50 | 11 micron |
| D90 | 19 micron |

### Example 3

### [1S-([1α(β*,δ*)2α,6α,8B(R*),8aα]]-1,2,6,7,8,8a-hexahydro-β,δ,6-trihydroxy-2-methyl-8-(2-methyl-1-oxobutoxy)-1-naphthalene heptanoic acid sodium salt (Amorphous Pravastatin)

A heterogenous mixture of pravastatin sodium (5g) was added to water (100ml) and the resulting reaction mixture was spray dried to afford amorphous pravastatin sodium. The amorphous pravastatin sodium so obtained was milled using an air jet mill. The particle size as measured by malvern particle size analyser gave the following pattern:

| | |
|---|---|
| D10 | 2 micron |
| D50 | 4 micron |
| D90 | 10 micron |

### Example - 4

### [R-(R*,R*)]-2-(4-fluorophenyl)-β,δ-dihydroxy-5-(1-methylethyl)-3-phenyl-4-[(phenylamino)carbonyl]-1H-pyrrole-1-heptanoic acid hemi calcium salt (Amorphous Atorvastatin calcium).

A heterogenous mixture of atorvastatin (5g) was added to methanol (100ml) and the resulting reaction mixture was spray dried to afford amorphous atorvastatin.

The particle size as measured by malvern particle size analyser gave the following pattern:

| | |
|---|---|
| D₁₀ | 1 microns |
| D₅₀ | 6 microns |
| D₉₀ | 11 microns |

### Example 5

### [1S-([1α(β*,δ*)2α,6α,8B(R*),8aα]]-1,2,6,7,8,8a-hexahydro-β,δ,6-trihydroxy-2-methyl-8-(2-methyl-1-oxobutoxy)-1-naphthalene heptanoic acid sodium salt (Amorphous Pravastatin)

A heterogenous mixture of pravastatin sodium (5g) was added to methanol (100ml) and the resulting reaction mixture was freeze dried to afford amorphous pravastatin sodium. The amorphous pravastatin sodium so obtained was milled using an air jet mill

The particle size as measured by malvern particle size analyser gave the following pattern:

| | |
|---|---|
| D10 | 2 micron |
| D50 | 8 micron |
| D90 | 16 micron |

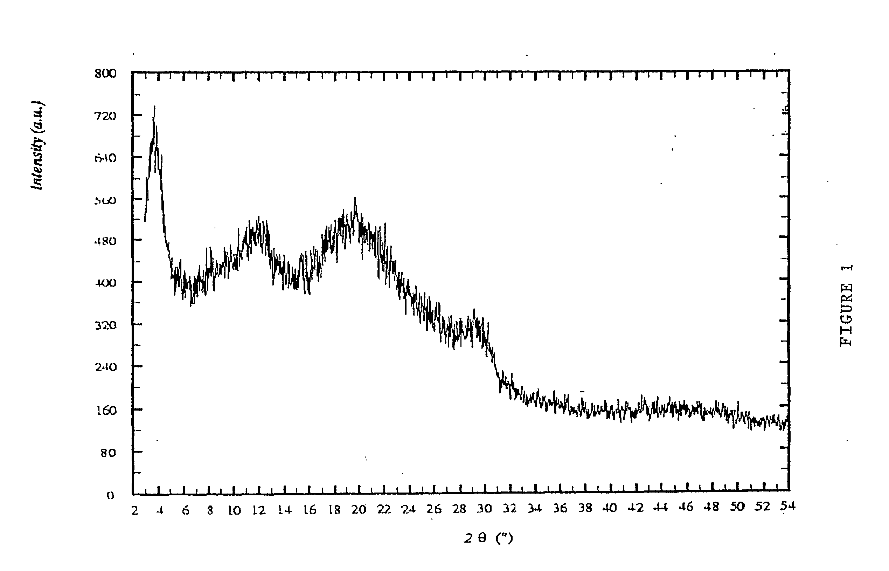

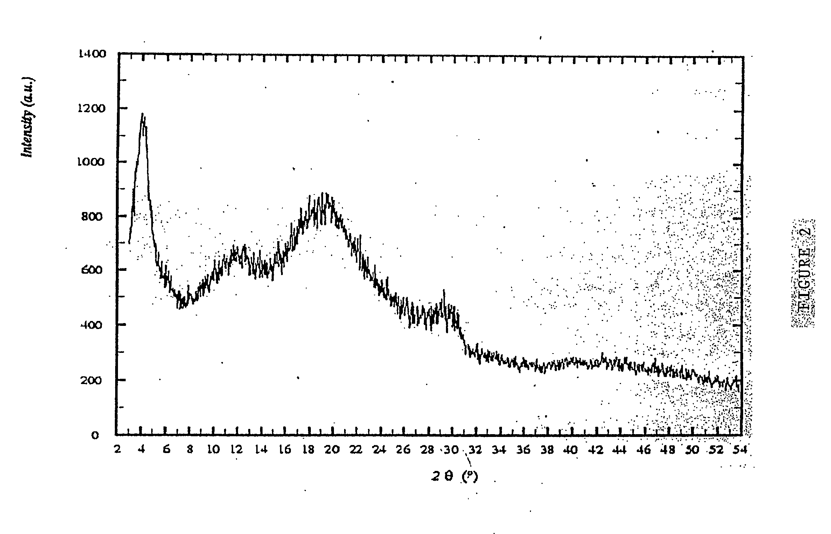

X-ray powder diffraction pattern (Figure 1 and 2 as shown in the accompanied drawings) demonstrates the amorphous nature of the product.

## Claims

1. A process for the preparation of amorphous atorvastatin Ca of formula I or amorphous pravastatin Na of formula II and hydrates thereof of desired particle size, 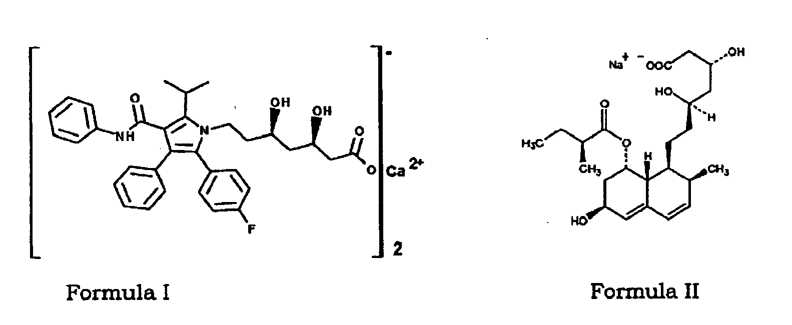 which comprises:
i. dissolving a mixture of amorphous and crystalline forms of either atorvastatin Ca or pravastatin in a hydroxylic solvent,
ii. removing the solvent to obtain either atorvastatin Ca or pravastatin by freeze drying

2. A process according to claim 1, further comprising
iii. subjecting the amorphous atorvastatin Ca or pravastatin Na to milling.

3. A process according to claim 1 or claim 2 wherein the amorphous atorvastatin Ca or pravastatin Na has a particle size of 1 to 150 microns.

4. A process according to any preceding claim wherein the hydroxylic solvent in step i is methanol.

## Patentansprüche

1. Verfahren für die Herstellung von amorphem Atorvastatin Ca der Formel I oder amorphem Pravastatin Na der Formel II und von Hydraten derselben von erwünschter Teilchengröße, 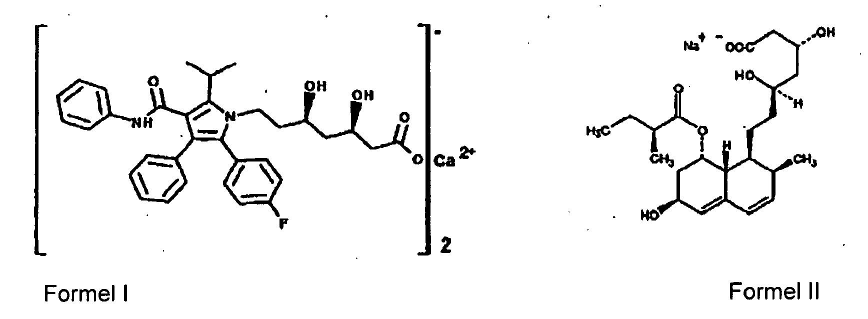 das Folgendes umfasst:
i. das Lösen einer Mischung von amorphen und kristallinen Formen von entweder Atorvastatin Ca oder Pravastatin in einem hydroxylischen Lösungsmittel,
ii. das Entfernen des Lösungsmittels durch Gefriertrocknen, um entweder Atorvastatin Ca oder Pravastatin zu erhalten.

2. Verfahren nach Anspruch 1, des Weiteren umfassend:
iii) das Unterwerfen des amorphen Atorvastatins Ca oder Pravastatins Na einem Mahlvorgang.

3. Verfahren nach Anspruch 1 oder Anspruch 2, wobei das amorphe Atorvastatin Ca oder Pravastatin Na eine Teilchengröße von 1 bis 150 Mikron aufweist.

4. Verfahren nach einem der vorhergehenden Ansprüche, wobei das hydroxylische Lösungsmittel in Schritt i.Methanol ist.

## Revendications

1. Procédé pour la préparation de Ca d'atorvastatine amorphe de formule I ou de Na de pravastatine amorphe de formule II et d'hydrates de ces derniers ayant la répartition granulométrique désirée, 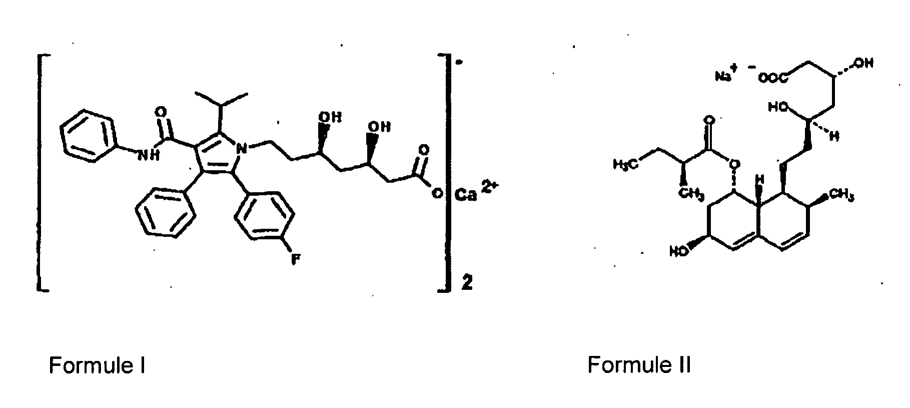 comprenant les étapes qui consistent à :
i. dissoudre un mélange de formes amorphes et cristallines soit de Ca d'atorvastatine, soit de pravastatine dans un solvant hydroxylique,
ii. extraire le solvant pour obtenir soit du Ca d'atorvastatine, soit de la pravastatine par lyophilisation.

2. Procédé selon la revendication 1, comprenant, en outre, l'étape qui consiste à
iii. soumettre le Ca d'atorvastatine amorphe ou le Na de pravastatine amorphe à un broyage.

3. Procédé selon la revendication 1 ou 2, dans lequel le Ca d'atorvastatine amorphe ou le Na de pravastatine amorphe a une répartition granulométrique de 1 à 150 microns.

4. Procédé selon l'une quelconque des revendications précédentes, dans lequel le solvant hydroxylique utilisé à l'étape i. est le méthanol.
